# EUROPÄISCHE PATENTSCHRIFT

(11) **EP 3 893 778 B1**
(45) Veröffentlichungstag und Bekanntmachung des Hinweises auf die Patenterteilung: **23.11.2022**
(21) Anmeldenummer: 20733926.8
(22) Anmeldetag: 16.06.2020
(51) Int. Cl.: A61B 17/70

(54) **TEILWEISE BLOCKIERTE PEDIKELSCHRAUBE**
PARTIALLY BLOCKED PEDICLE SCREW
VIS PÉDICULAIRE BLOQUÉE EN PARTIE

(30) Priorität: 17.06.2019 DE 102019116368
(43) Veröffentlichungstag der Anmeldung: 20.10.2021
(73) Patentinhaber: Aesculap AG, 78532 Tuttlingen (DE)
(72) Erfinder: WENZEL, Rudolf, 66620 Nonnweiler-Primstal (DE)
(74) Vertreter: Winter, Brandl - Partnerschaft mbB
(86) Internationale Anmeldenummer: PCT/EP2020/066605
(87) Internationale Veröffentlichungsnummer: WO 2020/254310

(56) Entgegenhaltungen:
- EP-A1- 1 570 796
- US-A1- 2010 036 436

## Beschreibung

Die vorliegende Offenbarung betrifft eine teilweise blockierte Pedikelschraube (kurz: Schraube) der polyaxialen Bauart mit einem Schraubenschaft und einem damit stoffeinstückig verbundenen Schraubenkopf, welcher drehbar und/oder verschwenkbar in einer Aufnahmehülse (Tulpe) gelagert bzw. aufgenommen ist.

### Hintergrund der Erfindung

Pedikelschrauben insbesondere der Polyaxialbauart werden bei chirurgischen Eingriffen an der Wirbelsäule verwendet, um mehrere Wirbel zueinander lagebestimmt festzulegen. Die Schrauben werden dazu jeweils in Pedikel eines Wirbels eingeschraubt und mittels einem in die Schraubenköpfe bzw. die damit gekoppelten Tulpen eingesetztem Verbindungsstab verbunden. Je nach Einsatzzweck kommen dabei Schrauben mit unterschiedlichen Freiheitsgraden bezüglich einer Relativbewegung zwischen einer Tulpe und einem Schraubenschaft zum Einsatz. Beispielsweise werden zur Derotation von deformierten Wirbelsäulen Schrauben vorgesehen, bei welchen eine Schwenkbewegung zwischen der Tulpe und dem Schraubenschaft in medial-lateraler Richtung blockiert ist (d.h. in seitlicher Richtung bezüglich einer Wirbelsäule bzw. des zugehörigen menschlichen Körpers oder in einer Transversalebene), in kranial-kaudaler Richtung (d.h. in Längsrichtung oder Höhenrichtung bezüglich der Wirbelsäule oder in einer Sagitalebene) jedoch zur Positionierung während der Operation zugelassen wird. Dies wird dadurch erreicht, indem der Schraubenkopf unrund, beispielsweise mit seitlich Abflachungen ausgebildet ist und ein in die Tulpe eingesetztes Inlay oder Insert zur axialen Druckbeaufschlagung des Schraubenkopfs für ein Fixieren der Relativlage zwischen Schraube und Tulpe mit ensprechenden seitlichen Innenflächen ausgebildet ist, die mit den Abflachungen am Schraubenkopf in Auflage kommen.

Derartige Pedikelschrauben werden mittels eines speziellen Instruments oder Instrumentensatzes implantiert, welches/welcher in der Regel für eine Anzahl von unterschiedlichen Pedikelschrauben standardisiert ist.

### Stand der Technik

Aus dem Stand der Technik, beispielsweise aus US 2010/0305621 A1 und US 9 138 261 B2, ist jeweils eine polyaxiale Pedikelschraube bekannt, deren runder, in einem Aufnahmekörper eingesetzter Kopf an zwei einander gegenüberliegenden Seiten zumindest teilweise abgeflacht ist, sodass eine proximal des Kopfes angeordnete Buchse (Inlay/Insert zum axialen Einsetzen in eine Tulpe) mit zwei ohrenförmigen axialen Vorsprüngen, welche flache Innenflächen ausbilden, mit diesen Innenflächen an den abgeflachten Seiten des Kopfs geführt sind und eine Verschwenkung des Schraubenschafts und -kopfs gegenüber der Buchse nur in einer Ebene parallel zu den flachen Innenflächen erlauben. Ein Teil des Kopfes der Pedikelschraube kann über die abgeflachten Seiten hinaus schraubenradial vorstehen, um eine zusätzliche schraubendiametrale Abstützung des Schraubenkopfs an dem Aufnahmekörper zu ermöglichen. Dies begrenzt jedoch einen erreichbaren Verschwenkungswinkel und verkleinert die Führungsfläche. Ferner sind die Flansche geringfügig elastisch deformierbar. Demgemäß können hohe Querkräfte ggf. nicht ausreichend abgestützt werden.

US 8 048 133 B2 offenbart eine polyaxiale Pedikelschraube, deren runder Kopf an zwei einander schraubendiametral gegenüberliegenden Seiten bearbeitet ist, um einen schraubendiametral verlaufenden Zylinder zu bilden, welcher proximal mit einer entsprechenden Fläche eines Druckelements (Inlay/Insert) in Anlage kommt und zusammen mit dieser Fläche eine Art Gleitlager bildet, welches Drehungen um eine Zylinderachse erlaubt und Drehungen/Schwenkbewegungen um andere Achsen blockiert. Dieses Blockieren einer Drehung oder Verschwenkung um andere Achsen ist jedoch bei hohen Querkräften voraussichtlich nicht ausreichend stabil und führt zu einem schnellen Ausleiern oder Abnutzen der Verbindung.

Ein weiteres Dokument, US 5 989 254 A, zeigt eine polyaxiale Pedikelschraube mit einem kugeligen Kopf, in welchem eine zylindrische Sattelfläche bzw. ein Querschlitz mit sattelförmig längsgekrümmtem Schlitzgrund zur Aufnahme einer Verbindungsstange, mittels welcher mehrere Pedikelschrauben verbindbar sind, ausgespart ist. In Fall, dass eine Tulpe über den Schraubenkopf der Pedikelschraube gesetzt und eine Verbindungsstand in die Tulpe quereingelegt ist, wälzt sich die Sattelfläche bei einer Verschwenkung des Schraubenkopfs in der Tulpe um eine schraubendiametral verlaufende Achse an der Verbindungsstange ab. Querkräfte werden hauptsächlich über eine Außenfläche der Verbindungsstange und Seitenflächen der Aussparung für die Sattelfläche, also über einen relativ kurzen Linienkontakt, übertragen. Diese Konstruktion ist gegenüber hohen Querkräften voraussichtlich nicht ausreichend stabil.

EP 1 570 796 A1 offenbart ein Knochenverankerungselement mit einem Schaft zur Verankerung in einem Knochen oder Wirbel, einem mit dem Schaft verbundenen ersten Kopfteil, einem das erste Kopfteil aufnehmenden zweiten Kopfteil, einem das zweite Kopfteil aufnehmenden Aufnahmeteil und einer Fixiervorrichtung zum Fixieren des zweiten Kopfteils derart, dass der Schaft relativ zu dem Aufnahmeteil in einer einstellbaren vorgegebenen Ruhewinkelstellung fixiert ist. Das erste und das zweite Kopfteil sind derart miteinander verbunden, dass der Schaft relativ zu dem zweiten Kopfteil aus der vorgegebenen Ruhewinkelstellung auslenkbar ist und dabei eine in die Ruhewinkelstellung rücktreibende Kraft wirkt. Nach einer Ausführungsform ist das erste Kopfteil zylindrisch mit einer umlaufenden Ausnehmung nach Art einer Spiralfeder ausgebildet.

Zusammenfassend lässt sich sagen, dass bekannte Lösungen zu viele Einzelteile der polyaxialen Pedikelschraube aufweisen und/oder Gelenke enthalten und somit aufwändig zu fertigen und zu montieren sind. Insbesondere ist häufig die Innengeometrie des proximal des Schraubenkopfs eingesetzten Inserts oder Einsetz- bzw. Haltestücks innerhalb der Tulpe aufwändig. Dies erhöht u.a. die Fertigungskosten. Ggf. ist für eine Implantation dieser Schrauben zudem ein eigenes Instrumentarium notwendig, was zugehörige Fertigungskosten aufgrund geringerer Stückzahlen weiter erhöht.

### Kurzbeschreibung der Erfindung

Der vorliegenden Erfindung liegt eine Aufgabe zugrunde, Nachteile des Stands der Technik zu reduzieren oder zu vermeiden. Insbesondere soll eine einfache, stabile Pedikelschraube mit einem Schraubenkopf bereitgestellt werden, der innerhalb einer Aufnahmehülse bzw. Tulpe in einer (einzigen) schraubendiametralen Richtung verschwenkbar und in einer anderen schraubendiametral Richtung lagefestgelegt ist. Ferner sollen mit der vorliegenden Pedikelschraube insbesondere das Instrumentarium und Madenschrauben zum Fixieren der Schraube, welche vorzugsweise mehrfach gelöst und angezogen werden können sollen, gleicher Systeme verwendbar sein. In anderen Worten sollen die grundsätzlichen Funktionen und die Außengeometrie der "normalen" (systemgleichen) polyaxialen Pedikelschraube beibehalten werden.

### Zusammenfassung der Erfindung

Die der Erfindung zugrunde liegende Aufgabe wird durch eine Pedikelschraube mit dem Merkmal des Anspruchs 1 gelöst. Vorteilhafte Ausgestaltungen sind Gegenstand der Unteransprüche und werden später genauer beschrieben.

Der Grundgedanke der vorliegenden Erfindung besteht im Wesentlichen darin, den in der Regel kugelartigen Schraubenkopf an seinem axial proximalen Scheitel vorzugsweise V-förmig zu spalten bzw. mit einem vorzugsweise V-förmigen Spalt auszubilden und das aus dem Stand der Technik per se bekannte Inlay (welches innerhalb einer Tulpe einen Axialdruck auf den Schraubenkopf ausübt und so den Kopf und die Tulpe mit-/gegeneinander verspannt) über einen vorzugsweise plattenförmigen Steg (Platte/Federzunge) mit der Pedikelschraube vorzugsweise stoffeinstückig zu verbinden, indem der Steg in Schraubenaxialrichtung am Inlay und im Spaltgrund am Schraubenkopf (stoffeinstückig) fixiert wird/ist. Auf diese Weise kann der Steg innerhalb des Spalts federelastisch hin- und her schwenken (wobei die Spaltflanken als Endanschlagflächen dienen) und dadurch das Inlay längs des Schraubenkopfumfangs (pendelartig) bewegt werden.

Anders ausgedrückt, wird die Aufgabe gelöst durch eine polyaxiale Pedikelschraube mit einer Aufnahmehülse bzw. Tulpe und einem Schraubenkopf, welcher einen in der Aufnahmehülse/Tulpe verschwenkbar (oder drehbar) eingelegten oder einlegbaren distalen Schwenkabschnitt (Kugelkopf der Pedikelschraube) und einen in die Aufnahmehülse/Tulpe lagefestgelegt eingelegten oder einlegbaren proximalen Halteabschnitt (Inlay) aufweist. Diese beiden Abschnitte sind über zumindest einen Steg/Platte/Zunge derart integral miteinander verbunden, insbesondere einteilig gefertigt, dass sie um eine quer zu einer Schraubenlängsachse (also schraubendiametral) verlaufende Schwenkachse zueinander verschwenkbar sind. Vorzugsweise kann der Steg elastisch und/oder plastisch verformbar sein, um die Verschwenkung des Schwenkabschnitts und des Halteabschnitts zu ermöglichen.

Ferner ist der zumindest eine Steg ein Materialabschnitt, welcher zwischen zwei in dem Schwenkabschnitt (Schraubenkopf) oder in dem Halteabschnitt (Inlay/Insert) ausgesparten Schlitzen oder Keilen (d. h., schlitz- oder keilförmigen Aussparungen) angeordnet ist. Dadurch kann ein gleichmäßiges Verschwenken der beiden Bauteile gewährleistet werden.

In anderen Worten ausgedrückt sind bei der erfindungsgemäßen Pedikelschraube der Halteabschnitt (Inlay) und der Schwenkabschnitt (Schraubenkopf) einteilig hergestellt (oder durch Schweißen/Löten/Kleben) miteinander stoffeinstückig verbunden, wobei die Verschwenkbarkeit durch einen Verbindungsabschnitt oder Steg gewährleistet wird, welcher zumindest abschnittsweise ausreichend schmal ausgebildet ist, um mit vertretbarem Kraftaufwand und in einem ausreichend großen Bereich für die Anwendung bei einer Wirbelsäulenoperation verformt/gebogen werden zu können.

Als bevorzugter Werkstoff für eine solche Pedikelschraube ist eine Titanlegierung vorgesehen. Dies vereinfacht sowohl die Herstellung als auch die Kommissionierung der Einzelteile und die Montage der Pedikelschraube. Folglich können Fertigungskosten gesenkt werden.

Der Steg kann mit dem Halteabschnitt (Inlay/Insert) und dem Schwenkabschnitt (Schraubenkopf) an verschiedenen Stellen verbunden sein und kann diverse Formen aufweisen, beispielsweise gerade, schlaufen- oder s-förmig. Je nach Form und Anordnung kann der Steg unterschiedliche Eigenschaften aufweisen und kann z.B. gleichmäßig biegsam, gemäß einem bestimmten Gradienten biegsam oder nur an zumindest einer definierten Stelle biegsam sein. Gleichermaßen kann der Halteabschnitt (Inlay) unterschiedlich geformt sein, um ein Verdrehen oder Verrutschen in der Aufnahmehülse/Tulpe zu unterbinden, wobei der Halteabschnitt (Inlay) in Schraubenlängsrichtung ggf. erst durch Einschrauben einer Verbindungs-/Querstange und einer Madenschraube in die Aufnahmehülse/Tulpe festgelegt wird. Bevorzugt entspricht die Außengeometrie des Halteabschnitts (Inlay) einer Außengeometrie eines Inserts/Inlays einer systemgleichen Standardpedikelschraube. Um die Verschwenkbarkeit zwischen der Aufnahmehülse/Tulpe und dem Schwenkabschnitt (Schraubenkopf) zu gewährleisten, können entweder der Schraubenkopf und/oder die Aufnahmehülse an einem Kontaktbereich derselben konkav und konvex gerundet sein.

Es hat sich als sinnvoll erwiesen, wenn ein Ende des zumindest einen Stegs, an welchem dieser integral mit dem Halteabschnitt (Inlay/Insert) oder dem Schwenkabschnitt (Schraubenkopf) verbunden ist, als Kipp- oder Knickgelenk dient. Ggf. kann hierzu der Steg an der Stelle, an welcher dieser abknicken/verkippen soll, um die Verschwenkung zu erzeugen, z.B. eine Einschnürung oder einen verkleinerten Querschnitt aufweisen, welche(r) eine Sollknickstelle bereitstellt. Dies ist insbesondere deshalb möglich, da das somit ausgebildete Gelenk (Filmscharnier) bei der Implantation der Pedikelschraube mittels einer Madenschraube in Einbauposition fixiert wird und nur bis zu diesem Zeitpunkt intakt und beweglich sein muss. Demgemäß ist kein Brechen des Stegs aufgrund von Dauerbelastung zu befürchten. Wenn wie vorstehend beschrieben in dem Schwenkabschnitt (Schraubenkopf) oder in dem Halteabschnitt (Inlay/Insert) Schlitze oder Keile ausgespart sind, ist dieses Kippgelenk bevorzugter Weise an dem Ende des Stegs vorgesehen, welches am Boden dieser Schlitze (Schlitzgrund) oder Keile angeordnet ist.

Zweckmäßigerweise dienen Flanken der Schlitze oder Keile als Anschlag zur Begrenzung der Verschwenkung des Schwenkabschnitts und des Halteabschnitts. Dadurch kann vorteilhafterweise die Verschwenkung des Halteabschnitts (Inlay/Insert) und des Schwenkabschnitts (Schraubenkopf) begrenzt werden, was insbesondere das Kippgelenk schont, wenn dieses zwischen den Schlitzen oder Keilen ausgebildet ist. Ferner wird dadurch ermöglicht, die Aufnahmehülse/Tulpe während einer Operation in einer definierten Anschlagsposition abzulegen. Die Schlitze oder Keile sollten dabei einen Verschwenkungswinkel von zumindest +/-22°, vorzugsweise zumindest +/-30° ermöglichen. D. h., falls die Keile oder Schlitze symmetrisch sind, betragen die Winkel zwischen der Schraubenlängsachse und den durch die Flanken gebildeten Anschlägen jeweils zumindest 22°, vorzugsweise zumindest 30°. Dies gewährleistet die notwendige Beweglichkeit der Pedikelschraube.

Bevorzugt sind die Schlitze oder Keile und/oder zwei Spalte, welche den Schwenkabschnitt (Schraubenkopf) und den Halteabschnitt (Inlay/Insert) trennen, durch ein Trennverfahren gefertigt bzw. herausgearbeitet, vorzugsweise durch Drahterodieren. In anderen Worten wird die Form, ohne sie zu teilen, durch Ausarbeiten von Keilen so verändert, dass sich der Halteabschnitt (Inlay/Insert), welcher die Funktion eines Inserts einer Standardpedikelschraube übernimmt, unter Verformung der Reststege bewegen lässt. Demgemäß kann der Pedikelschraubenkopf auf einfache Weise einstückig gefertigt werden, ohne die Kosten übermäßig zu erhöhen. Ferner werden hierdurch eine hohe Genauigkeit erzielt, insbesondere da demgemäß Flächen beiderseits des Schlitzes ohne zusätzliche Nachbearbeitung (ausreichend) genau zueinander passen.

Nach einer bevorzugten Ausgestaltung der Erfindung verläuft der zumindest eine Steg gerade. In diesem Fall ist der zumindest eine Steg verhältnismäßig steif und kann somit dazu beitragen, entsprechend hohe Querlasten aufzunehmen, wodurch er dazu beiträgt, ein Verschwenken um eine Achse quer zur Schwenkachse zu unterbinden. Ferner ist der zumindest eine Steg besonders einfach und somit kostengünstig zu fertigen. Alternativ kann der zumindest eine Steg auch gewellt ausgebildet sein. Dadurch können Vorteile hinsichtlich einer besseren Beweglichkeit des Stegs bzw. eine leichtere Verformung bei Bewegung des Halteabschnitts (und der Aufnahmehülse) erzielt werden.

Vorzugsweise hat der Schwenkabschnitt (Schraubenkopf) proximal eine Zylindermantelfläche und hat der Halteabschnitt (Inlay/Insert) distal eine korrespondierende Innenzylindermantelfläche. Alternativ hat der Schwenkabschnitt (Schraubenkopf) proximal eine Innenzylindermantelfläche und der Halteabschnitt (Inlay/Insert) distal eine korrespondierende Zylindermantelfläche. Diese komplementäre Form wird insbesondere durch das Einbringen eines Schlitzes erzielt und erlaubt eine Schwenk-/Drehbewegung des Halteabschnitts (Inlay/Insert) und des Schwenkabschnitts (Schraubenkopfs) zueinander. Insbesondere ist vorgesehen, dass das als Kippgelenk dienende Ende des Stegs sich auf einer Längsachse der Zylindermantelfläche befindet, um diese Drehung/Verschvenkung zu gewährleisten. Je nach Breite des Schlitzes und anliegender Belastung (insbesondere bei einem gewellten Steg) können diese beiden Flächen in Anlage kommen und als Führungsgeometrie dienen. Grundsätzlich kann der Schlitz jedoch auch so groß gefertigt sein, dass die beiden Flächen deutlich beabstandet sind, da eine Führungsgeometrie bei der erfindungsgemäßen Pedikelschraube grundsätzlich entfallen kann. In beiden Fällen sind der Halteabschnitt (Inlay/Insert) und der Schwenkabschnitt (Schraubenkopf) derart gestaltet, dass die Zylindermantelfläche und die Innenzylindermantelfläche einander in jeder Schwenkposition zumindest teilweise überlagern. Auf diese Weise kann ggf. eine Führungs- oder Stützfunktion (insbesondere im Einbauzustand) gewährleistet werden. Ferner werden dadurch die Keile oder Schlitze stets abgeschirmt, sodass kein Fremdkörper o.Ä. darin eingebracht wird und möglicherweise die Beweglichkeit des Schraubenkopfs blockieren kann.

Ferner hat es sich als vorteilhaft erwiesen, wenn der Schwenkabschnitt (Schraubenkopf) seitlich und proximal derart verbreitert ist, dass er zumindest teilweise einen in Schraubenlängsrichtung verlaufenden (Haupt-) Zylinder bildet, um sich an einer Innenfläche der Aufnahmehülse/Tulpe in Richtung der Schwenkachse abzustützen. Durch diese besonders stabile Ausgestaltung können hohe Seitenlasten von bis zu 500N abgestützt werden, um eine Verschwenkung um eine Achse quer zu der Schwenkachse zu blockieren. Vorzugsweise stützen sowohl diese zylinderförmige (Klemm-) Fläche als auch der zumindest eine Steg die Seitenlasten ab. In diesem Fall ist es sinnvoll, in der Aufnahmehülse/Tulpe (welche vorzugsweise dem Körper oder "Body" einer standardmäßigen Pedikelschraube entspricht) Aussparungen vorzusehen, welche vorzugsweise einen im Wesentlichen rechteckigen oder quadratischen Raum ausbilden, sodass die Pedikelschraube innerhalb der Aufnahmehülse/Tulpe beweglich ist. Insbesondere ist es notwendig, dass die Aussparungen derart ausgebildet sind, dass der zylindrische Teil (der Hauptzylinder) des Schwenkabschnitts in jeder Schwenkposition darin Platz findet.

Bevorzugterweise erlaubt die vorstehend beschriebene Pedikelschraube die Verschwenkung jeweils in einer kranial-kaudalen Richtung und sperrt vorzugsweise eine Verschwenkung in medial-lateraler Richtung. Insbesondere soll eine kranialkaudale Beweglichkeit des Schraubenkopfs unter Seitenlasten von mindestens 500N unterbunden werden. Diese Richtungsangaben nehmen Bezug auf eine vorgesehene Einbausituation der Pedikelschraube während einer Operation. D. h., wenn die Pedikelschraube in die Wirbelsäule eingesetzt wird, sollen der Schraubenschaft und die Aufnahmehülse in kranial-kaudaler Richtung, d. h. in einer Sagitalebene, zueinander beweglich sein und sollen in lateral-medialer Richtung, d. h., in einer Transversalebene, lagefestgelegt sein.

Es ist außerdem zweckmäßig, wenn ein durch den Halteabschnitt (Inlay/Insert) und in den Schwenkabschnitt (Schraubenkopf) verlaufender Kanal vorgesehen ist, um eine Werkzeugaufnahme zur Implantation der Pedikelschraube zu bilden. Ggf. wird der Steg dadurch geteilt, sodass beiderseits des Kanals jeweils ein schmalerer Steg stehen bleibt.

### Figurenbeschreibung

Nachfolgend wird die vorliegende Erfindung anhand bevorzugter Ausführungsformen beschrieben. Diese sind jedoch nur veranschaulichender Natur und sollen den Schutzumfang der vorliegenden Erfindung nicht einschränken.
Fig. 1 zeigt einen Kopf einer Pedikelschraube nach einer ersten Ausführungsform der vorliegenden Offenbarung.
Fig. 2 zeigt einen Längsschnitt und einen Querschnitt einer Aufnahmehülse nach der ersten oder der zweiten Ausführungsform der vorliegenden Offenbarung.
Fig. 3 dient der Veranschaulichung des Funktionsprinzips der Pedikelschraube nach der ersten Ausführungsform.
Fig. 4 zeigt einen Kopf einer Pedikelschraube nach einer zweiten Ausführungsform der vorliegenden Offenbarung.

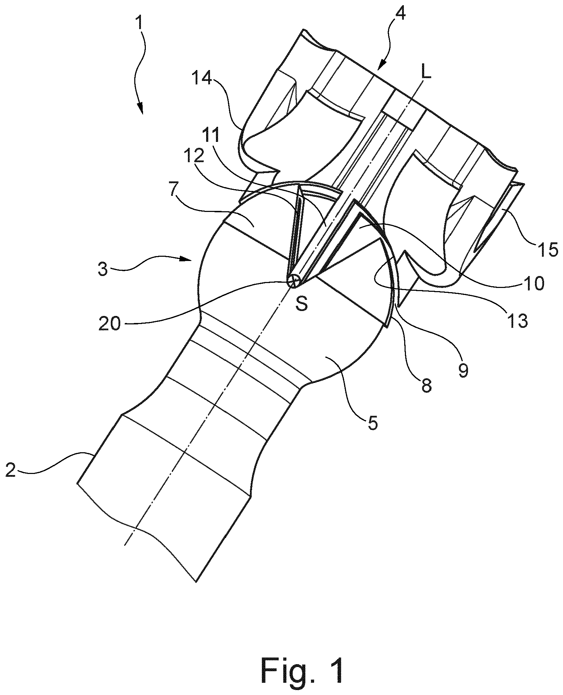

Fig. 1 zeigt einen Schraubenkopf 1 einer ersten Ausführungsform einer erfindungsgemäßen Pedikelschraube. Ein einstückig oder integral mit dem Schraubenkopf 1 ausgebildeter Schraubenschaft 2, welcher sich von dem Schraubenkopf 1 in distaler Richtung erstreckt, ist hier nur ansatzweise dargestellt.

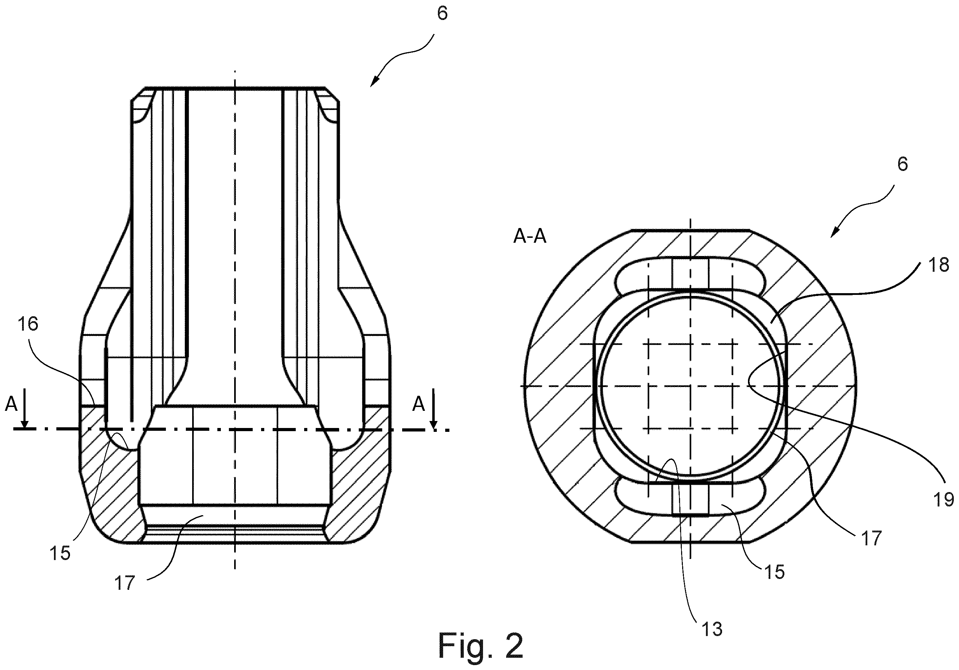

Der Schraubenkopf 1 ist zweiteilig ausgebildet und weist einen distalen Schwenkabschnitt 3 (entspricht dem eigentlichen Schrauben- oder Kugelkopf der Pedikelschraube am proximalen Ende des Schraubenschafts 2) und einen proximalen Halteabschnitt 4 (entspricht dem Insert/Inlay innerhalb einer Tulpe) auf. Der Schwenkabschnitt 3 ist zumindest an seinem an den Schraubenschaft 2 angeschlossenen distalen Ende 5 (vorzugsweise zumindest seiner distalen Hälfte) rund, vorzugsweise kugelförmig, ausgebildet. Dieses runde Ende ist im vollständig montierten Zustand in einer Aufnahmehülse bzw. Tulpe 6 (in Fig. 2 dargestellt) aufgenommen und ermöglicht es, den Schraubenkopf 1 in der Aufnahmehülse 6 drehbar zu lagern. Ein proximales Ende 7 des Schraubenkopfs 1 (vorzugsweise näherungsweise eine proximale Hälfte) bildet zumindest teilweise einen sogenannten "Steinmetz-Körper" aus. D. h., das proximale Ende 7 wird aus zwei sich schneidenden Zylindern gebildet: einem Hauptzylinder, welcher sich ausgehend von dem distalen Ende 5 in proximaler Richtung (in Schraubenlängsrichtung) erstreckt, und einem Nebenzylinder, welcher sich in Richtung einer Schwenkachse S erstreckt und eine Zylindermantelfläche oder Führungsfläche 8 zum Führen einer Schwenkbewegung zwischen dem Schwenkabschnitt 3 und dem Halteabschnitt 4 bereitstellt. Der Nebenzylinder, insbesondere die Führungsfläche 8, ist durch ein Einbringen zweier einander diametral gegenüberliegender Spalte 9 in den zunächst ungeteilten Schraubenkopf 1 gefertigt, beispielsweise durch Drahterodieren.

Zudem sind ausgehend von der proximalen Mantelfläche des Nebenzylinders zwei zueinander symmetrische, keilförmige Aussparungen (Keile) 10 mittig in dem Schraubenkopf 1 eingebracht, beispielsweise gemeinsam mit den Spalten 9 durch Drahterodieren gefertigt. Diese Keile 10 sind in proximaler Richtung geöffnet, um jeweils mit einem der Spalte 9 verbunden zu sein, und erstrecken sich in distaler Richtung derart, dass sie zur Schwenkachse S, insbesondere zu einem Mittelpunkt des runden Schraubenkopfs 1, hin zulaufen und dass zwischen den zwei Keilen 10 ein Steg 11, oder in diesem Beispiel zwei Stege 11, stehen bleibt bzw. bleiben. D. h., jeder Steg 11 erstreckt sich von der Schwenkachse S gerade in proximaler Schraubenlängsrichtung L, sodass beiderseits des Stegs 11 die keilförmigen Aussparungen oder Keile 10 in dem Schraubenkopf 1 ausgespart sind, wobei diese vorzugsweise das proximale Ende 7 vollständig durchlaufen und bis in das distale Ende 5 hineinreichen. Jedem Steg 11 gegenüberliegende Flanken 12 der Keile 10 stellen Anschläge zur Begrenzung der Schwenkbewegung dar. Um eine Verschwenkung von zumindest +/-22°, vorzugsweise zumindest oder bis zu +/-30°, zu ermöglichen, weisen entsprechend die Keile 10 einen Winkel von zumindest +/-22°, vorzugsweise zumindest oder bis zu +/-30°, auf. Es ist anzumerken, dass in diesem Beispiel zwei einander diametral gegenüberliegende Stege 11 vorgesehen sind, da in der dargestellten Pedikelschraube ein zentraler Kanal, beispielsweise zum Einpressen von Knochenzement und/oder zur Aufnahme eines Schraubinstruments zur Implantation der Schraube, bereitgestellt ist, welcher die beiden Stege 11 voneinander trennt. Alternativ kann jedoch auch nur ein einzelner, durchgehender Steg 11 bereitgestellt sein.

An einem proximalen Ende sind die Stege 11 integral mit dem Halteabschnitt 4 verbunden. Beiderseits der Stege 11 ist an einem distalen Ende des Halteabschnitts 4 entlang der Spalte 9 eine der Führungsfläche 8 des Schwenkabschnitts 3 gegenüberliegende, innenzylindermantelförmige Aufnahmefläche (Innenzylindermantelfläche) 13 ausgebildet. Deren Kontur folgt der der Führungsfläche 8 derart, dass die Spalte 9 eine konstante Breite aufweisen. D. h., die Führungsfläche 8 und die Aufnahmefläche 13 werden gefertigt, indem die Spalte 9 in den zunächst ungeteilten Schraubenkopf 1 eingebracht werden, um den Schwenkabschnitt 3 und den Halteabschnitt 4 voneinander zu trennen. Demgemäß ist der Halteabschnitt 4 an seiner distalen Seite zumindest teilweise zylindrisch ausgebildet, um den Hauptzylinder in Schraubenlängsrichtung L fortzusetzen. Vorzugsweise sind die Spalte 9 möglichst schmal, sodass die Führungsfläche 8 und die Aufnahmefläche 13 ggf. nach Art eines Gleitlagers zusammenwirken können.

Proximal der zylindrischen distalen Seite bildet der Halteabschnitt 4 zwei einander schraubendiametral gegenüberliegende Ausbuchtungen 14 aus, welche im Wesentlichen Teil eines Zylinders sind, der quer zu der Schwenkachse S verläuft, und sich anschließend in proximaler Richtung erstrecken. Diese Ausbuchtungen 14 sind dazu vorgesehen, um in einem korrespondierenden Sitz 15 der Aufnahmehülse 6 (siehe Fig. 2) aufgenommen und dort lagefestgelegt gehalten zu werden. Ferner ist der Halteabschnitt 4 längs der Ausbuchtungen 14 U-förmig geöffnet, um standardisierte Sattelflächen 16 zur Aufnahme einer Stange zum Verbinden mehrerer Pedikelschrauben bereitzustellen. Somit kann die erfindungsgemäße Pedikelschraube mit systemzugehörigem Instrumenten oder Zubehör wie einer solchen Stange verwendet werden. Gleiches gilt für die Ausgestaltung der Aufnahmehülse 6, genauer, deren Außenkonturen sowie ein darin eingelassenes Gewinde für Madenschrauben zum Festklemmen der Stange. Ferner ist anzumerken, dass durch die Anordnung der Ausbuchtungen 14 bezüglich des Stegs 11 die Richtung der Schwenkbewegung bei Implantation der Pedikelschraube festgelegt ist, da die Stange meist im Wesentlichen parallel zu der Wirbelsäule eines Patienten verläuft. In diesem Beispiel würde die Stange quer zu der Schwenkachse S verlaufen, weshalb die Schwenkbewegung des Pedikelschraubenkofs 1 und -schafts 2 in der Sagitalebene des Patienten ermöglicht wird und durch die Stege 11 sowie durch ein nachfolgend beschriebenes Zusammenwirken des Schraubenkopfs 1 und der Aufnahmehülse 6 eine Schwenkbewegung in der Transversalebene blockiert wird.

In dem links in Fig. 2 dargestellten Längsschnitt der Aufnahmehülse 6 ist neben dem bereits beschriebenen Sitz 15 und den Sattelflächen 16 auch gut zu erkennen, dass ein Innendurchmesser der Aufnahmehülse 6 sich an ihrem distalen Ende über eine kegelige Aufnahmefläche 17 verengt. Diese Aufnahmefläche 17 dient zur schwenkbaren bzw. drehbaren Lagerung oder Aufnahme des runden distalen Endes 5 des Schraubenkopfs 1. In dem rechts in Fig. 2 dargestellten Querschnitt der Aufnahmehülse 6 ist zudem ein in die Aufnahmehülse eingebrachter, im wesentlichen quadratischer Raum 18 dargestellt, welcher unmittelbar proximal der Aufnahmefläche 17 in die Aufnahmehülse 6 eingelassen ist und dessen Seitenwände 19 im Wesentlichen tangential zu der Aufnahmefläche 17 verlaufen. Diese Seitenwände 19 sind dazu ausgebildet, um an dem Hauptzylinder des proximalen Endes 7 des Schwenkabschnitts 3 zusammenzupassen/anzuliegen und sich daran abzustützen, um eine Schwenkbewegung quer zu der Schwenkachse S zu blockieren.

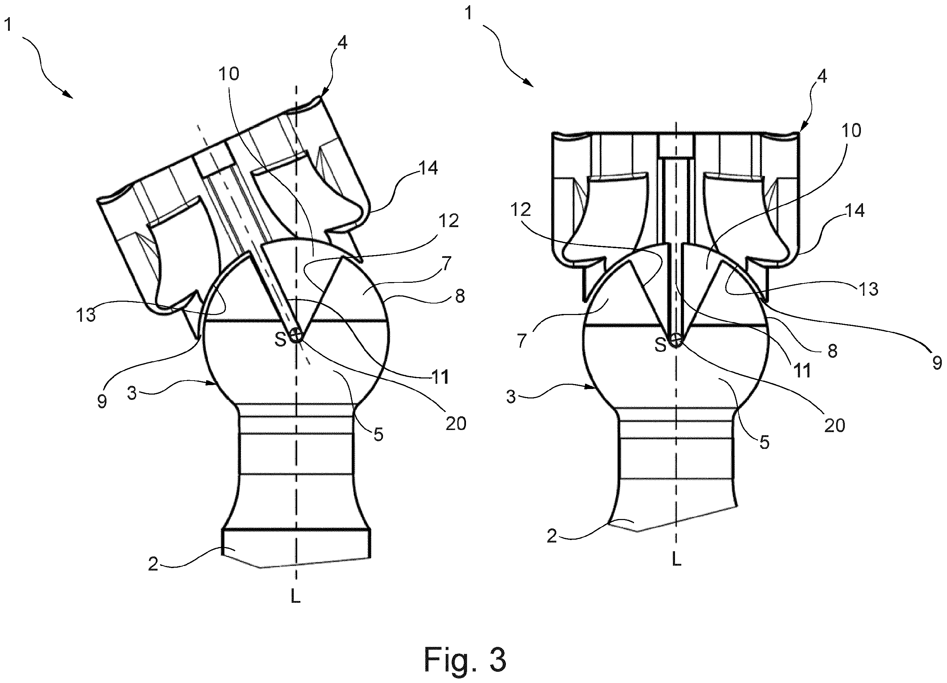

Fig. 3 dient der Veranschaulichung des Funktionsprinzips der erfindungsgemäßen Pedikelschraube. Die Darstellung des Schraubenkopfs 1 rechts entspricht im Wesentlichen der Darstellung aus Fig. 1, in welcher der Schwenkabschnitt 3 und der Halteabschnitt 4 nicht gegeneinander beschränkt sind (d. h., eine Längsachse des Halteabschnitt 4 der Schraubenlängsrichtung L entspricht). Gut zu erkennen ist zudem, dass die Führungsfläche 8 und die Aufnahmefläche 13 einander in Schraubenlängsrichtung überlappen. Links in Fig. 3 ist der Schraubenkopf 1 in einem maximal verschwenkten Zustand des Schwenkabschnitts 3 und des Halteabschnitts 4 dargestellt. Die Führungsfläche 8 und die Aufnahmefläche 13 erstrecken sich weit genug, dass diese sich auch in diesem maximal verschwenkten Zustand einander in Schraubenlängsrichtung überlappen und somit während der vollständigen Schwenkbewegung eine Führung bereitstellen. Der Steg 11 ist in diesem Zustand an seinem distalen Ende abgeknickt, d. h. zumindest elastisch, gegebenenfalls auch plastisch, verformt. Das distale Ende des zumindest einen Stegs 11 fungiert somit als eine Art Kippgelenk 20, um welches jeder Steg 11 um die Schwenkachse S kippt. Ferner ist der Steg 11 in dem maximal verschwenkten Zustand mit der Flanke 12 eines der Keile 10 in Anlage, wodurch ein maximaler Schwenk- oder Kippwinkel begrenzt wird. Dieser Keil 10 ist folglich durch die Verformung des Stegs 11 geschlossen und der gegenüberliegende Keil 10 ist näherungsweise doppelt so breit.

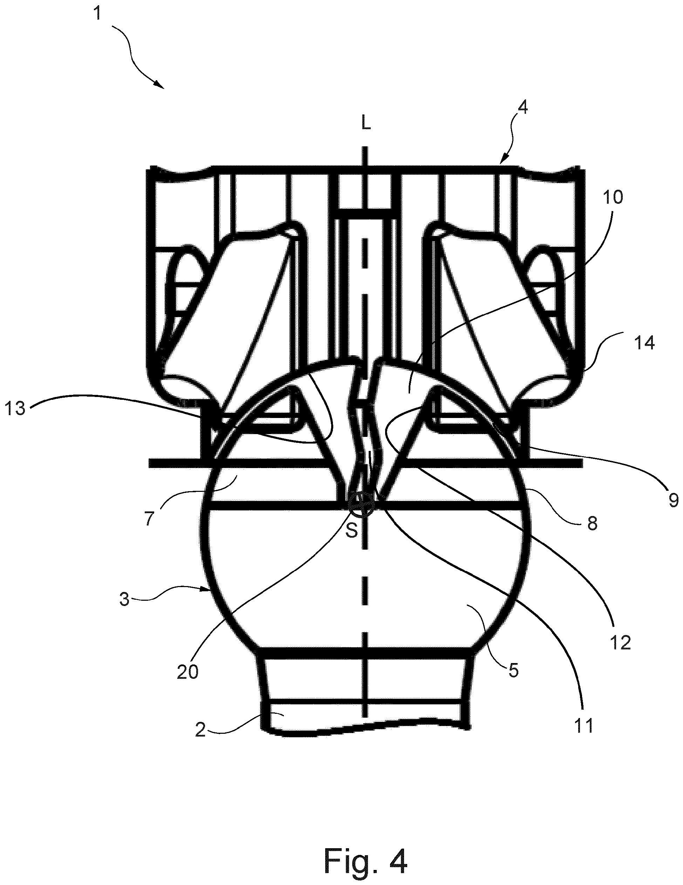

Fig. 4 ist eine Darstellung des Schraubenkopfs 1 nach einer weiteren Ausführungsform der vorliegenden Offenbarung. Diese Ausführungsform entspricht im Wesentlichen der ersten Ausführungsform, weshalb nachfolgend lediglich Unterschiede zwischen den beiden Ausführungsformen erläutert werden, wobei dieselben Bezugszeichen für einander entsprechende Merkmale verwendet werden. Die hier dargestellte Pedikelschraube nach der zweiten Ausführungsform hat, im Gegensatz zu dem geraden Steg 11 der ersten Ausführungsform, einen Steg 11, der gewellt ausgebildet ist. Der gewellte Steg 11 ist leichter verformbar, wodurch ein Ausrichten der Aufnahmehülse 6 über den Halteabschnitt 4 und den Steg 11 weniger Kraft seitens des Anwenders erfordert. Entsprechend wird auch weniger Kraft über den Schraubenschaft 2 auf das Gewebe übertragen und wird eine Handhabung während der Operation vereinfacht. Andererseits ist der Steg 11 auch gegenüber eine Verformung quer zu der Schwenkachse S leichter deformierbar, weshalb Querlasten gegenüber der ersten Ausführungsform vermehrt über den Hauptzylinder des proximalen Endes 7 des Schwenkabschnitts 3 aufgenommen werden müssen.

### Referenzzeichenliste

- 1: Schraubenkopf
- 2: Schraubenschaft
- 3: Schwenkabschnitt
- 4: Halteabschnitt
- 5: distales Ende des Schwenkabschnitts
- 6: Aufnahmehülse
- 7: proximales Ende des Schwenkabschnitts
- 8: Führungsfläche / Zylindermantelfläche
- 9: Spalt
- 10: Keil (keilförmig Ausnehmung)
- 11: Steg
- 12: Flanke
- 13: Aufnahmefläche / Innenzylindermantelfläche
- 14: Ausbuchtung
- 15: Sitz
- 16: Sattelfläche
- 17: kegelige Aufnahmefläche
- 18: Raum
- 19: Seitenwand
- 20: Kippgelenk

## Patentansprüche

1. Pedikelschraube mit einer Aufnahmehülse (6) und einem Schraubenkopf (1), welcher einen in der Aufnahmehülse (6) verschwenkbar einlegbaren oder eingelegten, vorzugsweise kugelförmigen distalen Schwenkabschnitt (3) und einen in die Aufnahmehülse (6) lagefestgelegt einlegbaren oder eingelegten proximalen Halteabschnitt (4) des Schraubenkopfs (1) aufweist, welche über zumindest einen vorzugsweise elastisch und/oder plastisch verformbaren Steg (11) derart integral miteinander verbunden sind, insbesondere einteilig gefertigt sind, dass sie um eine quer zu einer Schraubenlängsachse (L) verlaufende Schwenkachse (S) zueinander verschwenkbar sind, **dadurch gekennzeichnet, dass** der zumindest eine Steg (11) ein zwischen zwei in dem Schwenkabschnitt (3) oder in dem Halteabschnitt (4) ausgesparten Schlitzen oder Keilen (10) angeordneter Materialabschnitt ist.

2. Pedikelschraube nach Anspruch 1, wobei ein Ende des zumindest einen Stegs (11), an welchem dieser integral mit dem Halteabschnitt (4) oder dem Schwenkabschnitt (3) verbunden ist, als Kippgelenk (20) dient.

3. Pedikelschraube nach einem der Ansprüche 1 und 2, wobei Flanken (12) der Schlitze oder Keile (10) als Anschlag zur Begrenzung der Verschwenkung des Schwenkabschnitts (3) und des Halteabschnitts (4) dienen.

4. Pedikelschraube nach einem der Ansprüche 1 bis 3, wobei die Schlitze oder Keile (10) einen Verschwenkungswinkel von zumindest +/-22°, vorzugsweise zumindest +/-30°, ermöglichen.

5. Pedikelschraube nach einem der Ansprüche 1 bis 4, wobei die Schlitze oder Keile (10) und/oder zwei Spalte (9), welcher den Schwenkabschnitt (3) und den Halteabschnitt (4) trennen, durch ein Trennverfahren, vorzugsweise Drahterodieren, herausgearbeitet sind.

6. Pedikelschraube nach einem der vorstehenden Ansprüche, wobei der zumindest eine Steg (11) gerade verläuft.

7. Pedikelschraube nach einem der Ansprüche 1 bis 5, wobei der zumindest eine Steg (11) gewellt verläuft.

8. Pedikelschraube nach einem der vorstehenden Ansprüche, wobei der Schwenkabschnitt (3) proximal eine Zylindermantelfläche (8) hat und der proximale Halteabschnitt (4) distal eine korrespondierende Innenzylindermantelfläche (13) hat, oder der Schwenkabschnitt (3) proximal eine Innenzylindermantelfläche hat und der proximale Halteabschnitt (4) distal eine korrespondierende Zylindermantelfläche hat, welche einander entsprechend in jeder Schwenkposition zumindest teilweise überlagern.

9. Pedikelschraube nach Anspruch 8, wobei das als Kippgelenk (20) dienende Ende des Stegs (11) sich auf einer Längsachse der Zylindermantelfläche (8) befindet.

10. Pedikelschraube nach einem der vorstehenden Ansprüche, wobei der Schwenkabschnitt (3) seitlich und proximal derart verbreitert ist, dass er zumindest teilweise einen in Schraubenlängsrichtung (L) verlaufenden Zylinder bildet, um sich an einer Innenfläche der Aufnahmehülse (6) in Richtung der Schwenkachse (S) abzustützen.

11. Pedikelschraube nach einem der vorstehenden Ansprüche, wobei in der Aufnahmehülse (6) Aussparungen vorgesehen sind, welche vorzugsweise einen im Wesentlichen rechteckigen Raum (18) ausbilden, sodass die Pedikelschraube innerhalb der Aufnahmehülse (6) beweglich ist.

12. Pedikelschraube nach einem der vorstehenden Ansprüche, wobei die Verschwenkung jeweils in kranial-kaudaler Richtung ermöglicht wird und vorzugsweise in medial-lateraler Richtung gesperrt wird.

## Claims

1. Pedicle screw having a receiving sleeve (6) and a screw head (1), which has a preferably spherical, distal pivot section (3), which is pivotably insertable or inserted in the receiving sleeve (6), and a proximal holding section (4) of the screw head (1) which is insertable or inserted in a fixed position in the receiving sleeve (6), which are integrally connected to each other via at least one preferably elastically and/or plastically deformable web (11), in particular manufactured in one piece, in such a way that they are pivotable relative to each other about a pivot axis (S) running transversely to a longitudinal axis (L) of the screw, **characterized in that** the at least one web (11) is a material section arranged between two slots or wedges (10) recessed in the pivot section (3) or in the holding section (4).

2. Pedicle screw according to claim 1, wherein one end of the at least one web (11), at which the latter is integrally connected to the holding section (4) or to the pivot section (3) serves as a tilt joint (20).

3. Pedicle screw according to one of claims 1 and 2, wherein flanks (12) of the slots or wedges (10) serve as a stop for limiting the pivoting of the pivot section (3) and the holding section (4).

4. Pedicle screw according to one of claims 1 to 3, wherein the slots or wedges (10) allow a pivoting angle of at least +/-22°, preferably at least +/-30°.

5. Pedicle screw according to one of claims 1 to 4, wherein the slots or wedges (10) and/or two gaps (9), which separate the pivot section (3) and the holding section (4), are machined out by a separation process, preferably wire erosion.

6. Pedicle screw according to one of the preceding claims, wherein the at least one web (11) is straight.

7. Pedicle screw according to one of claims 1 to 5, wherein the at least one web (11) runs corrugated.

8. Pedicle screw according to one of the preceding claims, wherein the pivot section (3) proximally has a cylindrical lateral surface (8) and the proximal holding section (4) distally has a corresponding inner cylindrical lateral surface (13), or the pivot section (3) proximally has an inner cylindrical lateral surface and the proximal holding section (4) distally has a corresponding cylindrical lateral surface, which correspondingly at least partially overlap each other in each pivot position.

9. Pedicle screw according to claim 8, wherein the end of the web (11) serving as a tilt joint (20) is located on a longitudinal axis of the cylindrical lateral surface (8).

10. Pedicle screw according to one of the preceding claims, wherein the pivot section (3) is laterally and proximally widened in such a way that it at least partially forms a cylinder extending in the longitudinal direction (L) of the screw in order to bear against an inner surface of the receiving sleeve (6) in the direction of the pivot axis (S).

11. Pedicle screw according to one of the preceding claims, wherein recesses are provided in the receiving sleeve (6), said recesses preferably forming a substantially rectangular space (18), so that the pedicle screw is movable within the receiving sleeve (6).

12. Pedicle screw according to one of the preceding claims, wherein pivoting is enabled in each case in the cranial-caudal direction and is preferably locked in the medial-lateral direction.

## Revendications

1. Vis pédiculaire avec une douille de logement (6) et une tête de vis (1), laquelle présente une section de pivotement (3) distale, de préférence sphérique, qui peut être ou est insérée de façon à pouvoir pivoter dans la douille de logement (6) et une section de maintien (4) proximale de la tête de vis (1) qui peut être ou est insérée de façon solidement fixée dans la douille de logement (6), lesquelles sont reliées d'un seul tenant l'une à l'autre via au moins une barrette (11) qui peut être déformée de préférence élastiquement et/ou plastiquement, en particulier fabriquées en une pièce, de sorte qu'elles puissent être pivotées l'une par rapport à l'autre autour d'un axe de pivotement (S) qui se déroule transversalement à un axe longitudinal de vis (L), **caractérisée en ce que** la au moins une barrette (11) est une section de matériau agencée entre deux fentes ou cales (10) évidé(e)s dans la section de pivotement (3) ou dans la section de maintien (4).

2. Vis pédiculaire selon la revendication 1, dans laquelle une extrémité de la au moins une barrette (11), au niveau de laquelle celle-ci est reliée d'un seul tenant à la section de maintien (4) ou à la section de pivotement (3), sert d'articulation basculante (20).

3. Vis pédiculaire selon l'une des revendications 1 et 2, dans laquelle des flancs (12) des fentes ou cales (10) servent de butée pour limiter le pivotement de la section de pivotement (3) et de la section de maintien (4).

4. Vis pédiculaire selon l'une des revendications 1 à 3, dans laquelle les fentes ou cales (10) permettent un angle de pivotement d'au moins +/- 22°, de préférence au moins +/- 30°.

5. Vis pédiculaire selon l'une des revendications 1 à 4, dans laquelle les fentes ou cales (10) et/ou deux interstices (9), lesquels séparent la section de pivotement (3) et la section de maintien (4), sont façonnés par un procédé de séparation, de préférence un étincelage.

6. Vis pédiculaire selon l'une des revendications précédentes, dans laquelle la au moins une barrette (11) se déroule de façon rectiligne.

7. Vis pédiculaire selon l'une des revendications 1 à 5, dans laquelle la au moins une barrette (11) se déroule de façon ondulée.

8. Vis pédiculaire selon l'une des revendications précédentes, dans laquelle la section de pivotement (3) a proximalement une surface d'enveloppe de cylindre (8) et la section de maintien (4) proximale a distalement une surface d'enveloppe de cylindre intérieure (13) correspondante, ou la section de pivotement (3) a proximalement une surface d'enveloppe de cylindre intérieure et la section de maintien (4) proximale a distalement une surface d'enveloppe de cylindre correspondante, lesquelles se superposent au moins partiellement l'une à l'autre de façon correspondante dans chaque position de pivotement.

9. Vis pédiculaire selon la revendication 8, dans laquelle l'extrémité de la barrette (11) qui sert d'articulation basculante (20) se trouve sur un axe longitudinal de la surface d'enveloppe de cylindre (8).

10. Vis pédiculaire selon l'une des revendications précédentes, dans laquelle la section de pivotement (3) est latéralement et proximalement élargie de sorte qu'elle forme au moins partiellement un cylindre qui se déroule dans la direction longitudinale de vis (L) pour s'appuyer contre une surface intérieure de la douille de logement (6) dans la direction de l'axe de pivotement (S).

11. Vis pédiculaire selon l'une des revendications précédentes, dans laquelle dans la douille de logement (6) sont prévus des évidements, lesquels forment de préférence un espace (18) essentiellement rectangulaire, de sorte que la vis pédiculaire soit mobile à l'intérieur de la douille de logement (6).

12. Vis pédiculaire selon l'une des revendications précédentes, dans laquelle le pivotement est respectivement rendu possible dans la direction crânio-caudale et de préférence verrouillée dans la direction médio-latérale.
